# EUROPEAN PATENT APPLICATION

(11) **EP 4 459 275 A2**
(43) Date of publication of application: **06.11.2024**
(21) Application number: 24170034.3
(22) Date of filing: 12.04.2024
(51) Int. Cl.: G01N 27/66

(54) **PHOTOIONIZATION DETECTOR**

(30) Priority: 14.04.2023 GB 202305527
(71) Applicant: Alphasense Limited, Great Notley Essex CM77 7AA (GB)
(72) Inventor: BARON, Ronan, Braintree, CM77 7AA (GB); BENDZALA, Jakub, Braintree, CM77 7AA (GB); DUDENEY, Richard Henry, Braintree, CM77 7AA (GB); HACKETT, Paul Thomas Bramwell, Braintree, CM77 7AA (GB)
(74) Representative: Hindles Limited

(57) **Abstract**

A photo-ionization detector (PID) comprising: a UV source; an ionization chamber for receiving sample gas; a plurality of electrodes, forming an electrode stack module, for detecting gaseous analyte ionized in the ionization chamber; a controller; and at least one sensor in electronic communication with the controller for measuring a condition of the sample gas. The electrode stack module comprises a memory.

## Description

### Field of the invention

The invention relates to the field of photoionization detectors for detecting volatile organic compounds in a gaseous medium.

### Background to the invention

A photoionization detector (PID) is known in the art. This is a gas detector, typically used to detect volatile organic compounds (VOCs) or other compounds. A PID uses radiation, typically in the UV range, to ionize the species of interest. A PID enables gases to be detected in concentrations down to parts per billion (ppb) levels.

### Summary of the invention

In a first aspect of the present invention there is provided a photo-ionization detector (PID) comprising: a UV source; an ionization chamber for receiving sample gas (typically by diffusion); a plurality of electrodes (typically an electrode stack), including a first electrode, for detecting gaseous analyte ionized in the ionization chamber; a controller; and at least one sensor in electronic communication with the controller for measuring at least one condition of the sample gas. Typically, the plurality of electrodes, includes the first electrode. Typically the PID comprises a sensor housing.

The first electrode may be a measurement electrode. The first electrode may be an anode. The first electrode may be a cathode.

Each of the UV source, the ionization chamber for receiving sample gas (typically by diffusion), the plurality of electrodes, including the first electrode, for detecting gaseous analyte ionized in the ionization chamber, the controller, and the at least one sensor in electronic communication with the controller for measuring at least one condition of the sample gas is typically housed within the sensor housing.

In an additional aspect the invention discloses a method of operating a photo-ionization detector (PID), wherein the PID comprises a UV source and a first electrode for generating a measurement signal, in which method a feedback signal compensates the measurement signal. It may be that the feedback signal originates from the first electrode and regulates the UV source. It may be that the feedback signal is obtained from a sensor for measuring at least one condition of the sample gas and compensates the measurement signal.

In a second aspect of the present invention there is provided a method of controlling a photo-ionization detector (PID), in which method a sample gas enters (typically diffuses) into an ionization chamber of the PID; said sample gas in the ionization chamber is irradiated by UV radiation from a UV source; a first electrode generates a first signal indicative of detected gaseous analyte; at least one sensor measures at least one condition of the sample gas and generates at least one sensor signal; a controller receives the first signal and the at least one sensor signal, Typically, the controller outputs a measurement signal determined from the first signal and compensated using the at least one sensor signal. It may be that the controller outputs a measurement signal compensated using data, for example data stored in the memory of the electrode stack module. This data may include calibration, linearisation, scaling, offset and compensation coefficients for the at least one sensor signal. Typically the PID comprises a plurality of electrodes, which may take the form of an electrode stack. The first electrode is typically one of the plurality of electrodes. The gaseous analyte is present in the sample gas. The detected gaseous analyte may be a single chemical species but more usually the detected gaseous analyte comprises a plurality of chemical species. The detected gaseous analyte typically comprises volatile organic compounds (VOCs).

By providing a sensor for measuring sample gas condition, the measurement response can be compensated, resulting in more accurate measurements. Variations in the response of the PID which occur due to condition of the sample gas are thereby compensated for.

The first signal is typically a current signal. The first electrode is typically maintained at a stable potential in use.

Typically, the electrode stack comprises an second electrode in addition to the first electrode. The electrode stack may additionally comprise a guardelectrode, typically situated between the second electrode and the first electrode. The second electrode may be a cathode. The second electrode may be an anode.

The second electrode and first electrode may be positioned within the ionization chamber. The guard electrode, if present, may be positioned within the ionization chamber. The guard electrode may be positioned between the second electrode and first electrode.

The second electrode, first electrode and, if present, guard electrode may be arranged to allow passage of radiation produced by the UV source.

The electrode stack may be in the form of a replaceable electrode stack module in the PID. That is to say, the first electrode, the second electrode, and the guard electrode if utilised, may form part of a single unit, which may be removed from the PID and replaced with a new unit as required. The PID may comprise a body and a replacement electrode stack module which is demountably attached to the body. The method may comprise removing the replaceable electrode stack module and replacing it with another electrode stack module.

The replaceable electrode stack may comprise the at least one sensor. The at least one sensor typically comprises a humidity and/or a temperature sensor. Thus the at least one sensor may be part of and so replaceable as a unit with the replaceable electrode stack.

The at least one condition of the sample gas is typically independent of the concentration of the detected chemical. The at least one condition of the sample gas typically comprises the temperature and/or humidity of the sample gas. The at least one sensor typically comprises a temperature sensor. The at least one sensor typically comprises a humidity sensor. The at least one sensor may comprise an integrated temperature and humidity sensor. The method typically comprises compensating for the temperature of the sample gas. The method typically comprises compensating for the humidity of the sample gas.

The temperature and/or humidity sensor may be positioned in a chamber which is separate to but in fluid communication with the ionization chamber. The PID may have a first inlet through which the ionization chamber communicates with sample gas adjacent the PID, typically by diffusion, and a separate second inlet through which the temperature and/or humidity sensor communicates with sample gas adjacent the PID, typically by diffusion. The first inlet and the second inlet may connect to two separate spaces within the PID. The two separate spaces within the PID may be fluidically connected to each other. The second inlet may be within the PID and fluidically connected to the sample gas through the first inlet. The first inlet and the second inlet may be one and the same. Signals representing each of the temperature and/or the humidity of the gas being sensed are typically passed from the temperature and/or humidity sensor to the controller.

The replaceable electrode stack module may comprise a controller. The controller of the replaceable electrode stack module may be provided in addition to the controller of the PID. The controller of the replaceable electrode stack module may be the controller of the PID. Thus the controller may be part of and so replaceable as a unit within the replaceable electrode stack.

The replaceable electrode stack module may comprise a memory, typically a solid-state memory. Thus the memory may be part of and so replaceable as a unit with the replaceable electrode stack module. Usefully, the memory may therefore store data concerning the (specific) replaceable electrode stack, for example calibration data, type data, identification data or usage data. The controller of the PID sensor may be operable to write data to the memory of the replaceable electrode stack module. This enables at least one parameter of the amount of use made of the replaceable electrode stack to be stored with the replaceable electrode stack module. The at least one parameter of the amount of use may for example be an amount of time for which the replaceable electrode stack module has been fitted to the PID sensor, or the amount of time for which the replaceable electrode stack has been used, or the amount of VOCs detected, for example the amount of each of a plurality of different VOCs, or another parameter indicative of the amount of fouling of or reduction in lifetime or efficiency of the replaceable electrode stack module to be stored. Information such as this may be utilised to give an indication of contamination build up, enabling the user to determine when an electrode stack should be replaced. This data is also useful if the replaceable electrode stack module is later to be checked, audited, replaced or reused with another PID as it enables usage logging to be monitored across multiple PIDs.

The replaceable electrode stack module may comprise the UV source. Thus, the UV source may be replaced with the replaceable electrode stack module. The optical window (described below) may be part of the replaceable electrode stack module. Thus, the optical window, which typically fouls in use, may be replaced, optionally along with the UV source, when the electrode stack module is replaced.

The UV source may be a replaceable item. The UV source may be removable from the PID after removal of the PID stack, for example.

The replaceable electrode stack module may comprise a UV monitor.

The replaceable electrode stack module may comprise a stack interface connector to connect the first electrode, the second electrode, the guard electrode if present. The stack interface connector may also provide connections which are required to any other element which forms part of the replaceable electrode stack, for example the at least one sensor, the memory, the UV source or a UV monitor where these are part of the replaceable electrode stack.

The controller may comprise a microprocessor or microcontroller. The PID may comprise a memory, for example a solid-state memory. The memory may be part of the controller, or it may be separate from the controller. The controller may comprise a plurality of different components in electronic communication with each other. A microprocessor or microcontroller may take several forms, for example an application specific integrated circuit (ASIC), a programmable system on a chip (PSoC), a field programmable gate array (FPGA), or a complex programmable logic device (CPLD).

The PID may output a digital measurement signal based on the first signal, and typically compensated taking into account the at least one sensor signal (e.g. compensated for sample gas temperature and/or humidity). The PID may comprise an analogue to digital converter to convert the current from the first electrode (the first signal) into a digital signal. The PID may comprise a digital to analogue converter to convert a calculated output signal from digital to analogue for output as an analogue measurement signal. The PID may output both an analogue measurement signal and a digital signal (e.g. digital measurement signals or other digital data, such as identification information, usage data or calibration data).

The PID may comprise a plurality of electrical connections (typically pins) for outputting an analogue measurement signal and a plurality of separate electrical connections (typically pins) for outputting a digital measurement signal. Thus, the PID may be usable with existing circuits which interface with an analogue interface and process analogue measurement signals but may provide additional functionality using digital signals through a digital interface. The method may comprise using the PID sensor through an analogue interface which communicates with the electrical connections for outputting an analogue measurement signal. The method may comprise using the PID sensor through a digital interface which communicates with the electrical connections for outputting a digital measurement signal.

The invention extends to a method of replacing a PID sensor comprising the steps of using a first PID sensor which does not comprise a digital interface through an analogue interface and then subsequently removing the first PID sensor from the analogue interface and fitting a second PID sensor, the second PID sensor being a photo-ionization detector according to the first aspect of the invention, and comprising a plurality of electrical connections (typically pins) for outputting an analogue measurement signal and a plurality of separate electrical connections (typically pins) for outputting a digital measurement signal, to both the analogue interface and also a digital interface, and using the digital interface to communicate with the second PID sensor.

The specific arrangement of the first electrode, second electrode and guard electrodes is typically dependent on factors such as which analyte gas or gases are of interest and in which range of sensitivity detection is desired. It is possible to have different replaceable electrode stack modules with different electrode arrangements, but with a common design of interface for connecting to the rest of the PID. For example, a replaceable electrode stack may comprise a guard electrode. An alternative replaceable electrode stack may comprise no guard electrode. A replaceable electrode stack may have a guard electrode which is positioned equidistant between the first electrode and the second electrode. A replaceable electrode stack may have a guard electrode which is positioned closer to the first electrode than to the second electrode. A replaceable electrode stack may have a guard electrode which is positioned closer to the second electrode than to the first electrode. A replaceable electrode stack may have electrodes whose surfaces are made of an inert metal, such as one of ruthenium, rhodium, palladium, osmium, iridium, platinum, gold, silver, or copper. The surface of an electrode may be of a material passivated against corrosion, such as stainless steel. The surface of an electrode of a replaceable electrode stack may be an alloy of one or more of ruthenium, rhodium, palladium, osmium, iridium, platinum, gold, silver, or copper. The second electrode and first electrode of an electrode stack in a PID typically define a drift chamber within the PID. The ionization chamber of the PID may coincide with the drift chamber of the electrode stack. The electrodes of the electrode stack are typically configured to allow passage of UV radiation from the UV source. A replaceable electrode stack may have electrodes which are of cylindrical form. A replaceable electrode stack may have electrodes which are of planar form. Any of the electrodes of a replaceable electrode stack may be in the form of a metallic grid.

The distance between the first electrode and the second electrode of a replaceable electrode stack may vary from 0.1 m to 10 mm. A replaceable electrode stack module typically comprises electrodes separated and held in place in respect of each other by an insulating material. Examples of the insulating material of a replaceable electrode stack include fluorinated polymers, such as PTFE (polytetrafluoroethyene) or PVDF (polyvinylidene fluoride), glass and ceramic , such as aluminium oxide or silicon oxide.

In a configuration in which the replaceable electrode stack comprises a replacement UV source, a (solid state) memory within the replaceable electrode stack may also store data relevant and specific to the UV source. For example, the memory may store electrical parameters for igniting and maintaining the UV source. The memory may contain a historic log of the operation life of the UV source. This information may be useful as the parameters required to operate the UV source may vary as the source ages. The electrical parameters required to strike the UV source may depend on the history of the source. The electrical parameters required to maintain the UV source in operation may vary over its lifetime. Furthermore, the electrical conditions required to strike the UV source and to maintain the UV source may be dependent on temperature. Temperature information obtained from the temperature sensor may be utilised to obtain the appropriate electrical conditions to strike and/or to maintain the UV source.

Temperature variation data may be stored in memory and the controller may be configured to vary the electrical conditions to strike and/or to maintain the UV source dependent on the stored temperature variation data. The stored temperature variation data may result from a calibration. The temperature variation data may be stored from historical data. The stored temperature variation data may be stored in the form of a table. The stored temperature variation data may comprise one or more of a functional relationship between a strike voltage value and ambient temperature, a functional relationship between a maintenance voltage value and ambient temperature. For example, it may be that the voltage required to strike the UV source may increase or decrease as the ambient temperature increases. It may also be that the voltage required to maintain the UV source in an excited state, i.e. in a state in which a steady discharge is maintained and a steady production of UV photons is produced, may increase or decrease as the ambient temperature increases. The memory may store information of temperature dependent voltages. Such information may be stored for example in the form of a table, or in the form of a functional relationship, resulting, for example, from a calibration procedure.

The PID may additionally comprise a driver circuit for driving the UV source and a driver circuit for providing the bias or biases for the first electrode, second electrode and guard electrode if present. The driver circuit for driving the UV source and the driver circuit for providing the bias or biases may be a single driver circuit or they may have features in common. The driver circuit or driver circuits may connect to the elements of the electrode stack using the stack interface connector. The driver circuit or driver circuits may comprise computer program code stored on a computer readable solid-state medium.

The UV source typically comprises an optical window through which UV radiation produced by the source emits. The UV radiation emitted thorough the optical window is directed towards the ionization chamber. A lamp cavity may be provided in the PID, within which the UV source may sit. The lamp cavity may serve to retain heat generated by the UV source within the lamp cavity. In other words, the lamp cavity may act as a heat shield protecting the PID elements from heat generated by the UV source.

The PID may comprise a sensor for detecting electromagnetic radiation emitted by the UV source. This electromagnetic radiation may be the UV radiation emitted through the optical window, but need not be this UV radiation. The electromagnetic radiation may be radiation emitted through a side wall of the UV source. The lamp cavity may comprise a window to allow this radiation to pass to the sensor for detecting electromagnetic radiation, which is suitably positioned to detect the radiation.

The basic operational principle of the PID is that UV radiation from the UV source photoionizes the analyte species to be measured, in the ionization chamber. The analyte species to be measured is typically a gaseous analyte typically comprising volatile organic molecules (VOCs). Bias voltages applied to the electrode stack create an electric field between the first electrode and the second electrode. Positive ions created through photoionization drift under the influence of the electric field to the first electrode where they are captured, creating a measurement signal. A guard electrode may be used to ensure that the measurement signal is protected from contributions due to other current sources. For example, a guard electrode placed between the first electrode and second electrode, may be biased with a positive potential to attract photoelectrons.

Typically, the measurement signal from the first electrode feeds into the controller, possibly after amplification by an amplifier. The signal from the sensor for measuring gas condition, typically being a humidity and/or temperature sensor, may also feed into the microcontroller. The microcontroller may be programmed to generate a gas concentration output from the measurement signal which is compensated for the gas conditions, typically humidity and temperature. The compensation of the measurement signal to account for the effects of typically temperature and humidity may be achieved through calibration. Calibration data, typically in the form of a calibration table, or a calibration curve, may be stored in a memory of the controller. Typically data stored in a memory may be rewritten, for example to bring it up to date. Calibration data may be stored in a non-volatile memory, for example a non-volatile memory associated with the electrode stack. A memory associated with an electrode stack may be a memory integrated in the electrode stack unit. A memory integrated in (or integrable in) an electrode stack may store data about the stack. Such data may include calibration data, which may be programmed during final testing of the stack at manufacture.

It may be that the operation of the PID is controlled by the controller, responsive to data stored in the memory of the PID. Where the electrode stack module comprises a memory, it may be that the operation of the PID is controlled by the controller, responsive to data stored in the memory of the electrode stack module.

The operation of the PID may be controlled by the controller responsive to sensor data from the at least one sensor.

The operation which is controlled may comprise controlling or setting the potentials of one or more of the electrodes in the electrode stack.

The operation which is controlled may comprise operation of the UV source.

The UV source may be in the form of a plasma lamp. A gas, typically a noble gas or a gas comprising a noble gas, is contained in a closed bulb where it is excited into a plasma state using radio frequency power supplied by electrodes placed at the exterior of the bulb.

The power necessary to ignite or to strike the UV source and the power necessary to maintain the plasma is dependent on temperature. The measured temperature (from the temperature sensor) of the PID may be processed by the controller, which may then use this information to adjust the power supplied to the UV source.

The UV source acts as an element in the electrical circuit used to power it. The electrical properties of the UV source in this circuit, such as its capacitance, vary as the UV source ages. This may result in a loss of efficiency due to the lamp no longer being optimally driven. The controller may regulate control of the UV source, for example to increase the power to the UV source over time to compensate for fouling of the electrodes in the electrode stack and/or the optical window.

The present disclosure envisages a procedure to optimise the signal powering the UV source. The procedure may also be used to find the optimal signal for powering the UV source, if this is not yet known.

The controller may be configured to switch the UV source (typically plasma lamp) repetitively on and off and may regulate the duration of each on period and the time between each on period. Thus, the PID may have an intermittent mode, typically in addition to a continuous operation mode in which the UV source remains on. The choice of mode may be made automatically or in response to a received user command. The proportion of time for which the UV source is on may be reduced by the controller to reduce power consumption or to extend source lifetime.

The UV source may be in a maintenance phase or in an inactive state. A maintenance phase of the UV source is a state in which the UV source may be on, that is producing UV radiation, or may be capable of being switched on. An inactive state is a state in which the UV source is off, is not producing UV radiation, and from which inactive state the UV source must be switched to an active state, or maintenance state, before it starts to produce UV radiation.

The controller may be configured to reduce the proportion of time for which the UV source is on responsive to measurements of VOC concentration (e.g. the first signal or the first signal, after compensation), for example responsive to a measurement of VOC concentration exceeding one or more thresholds. Fouling takes place more quickly at higher VOC concentrations. Fouling may take place more quickly when UV radiation is present. Thus, the proportion of time for which the UV source is on, and potentially the intensity of the UV light from the source, may be reduced when the VOC concentration exceeds a threshold to increase the lifetime of the electrode stack and/or PID sensor.

The controller may be configured to regulate the power to the UV source, when it is on, to switch it between a plurality of different power levels in a cycle. This may be combined with switching the UV source on and off and regulating the duration of each on period and the time between each on period. [Claim 17] The plurality of power levels typically comprises a strike phase, followed by at least one illumination phase. A higher power is required during the strike phase to switch the UV source on (at least when the UV source is a plasma lamp) but this power need not be maintained, thereby saving power.

Due to the relatively high power required to strike a plasma lamp UV source it is surprising that it is possible to save energy or increase the lifetime of the lamp by a process including switching the UV source on and off repetitively.

The controller may be configured to vary the frequency of the current driving the UV source (particularly when the UV source is a plasma lamp) during the strike phase, for example to sweep the frequency of the current driving the UV source, to facilitate finding the optimum frequency, typically the resonant frequency of the electrical circuit which includes the UV source. The optimum frequency can vary as the UV source ages and with parameters such as temperature and humidity. The controller may be configured to detect the striking of the UV source, for example by measuring the emitted light, and to change the frequencies (e.g. swept frequency range) for subsequent strikings of the UV lamp, for example using feedback control. The controller may be configured to monitor the drive current passing through the UV source as the frequency of the current is varied in an initial frequency sweep, in particular to monitor the frequency at which the resonant frequency of the circuit occurs, typically when the drive current passes through a minimum. The controller may also be configured to perform a periodic frequency sweep in the vicinity of, and typically through, the resonant frequency and to monitor the drive current passing through the UV source. In this way changes to the resonant frequency as the UV source ages can be followed. The frequency at which the UV source is driven may be maintained at the optimum frequency.

The at least one condition of the sample gas is typically the humidity and/or temperature of the sample gas. The at least one sensor is typically a humidity and/or temperature sensor.

The controller, which may comprise a microprocessor, typically generates the measurement signal prior to output by adjusting the first signal according to the at least one sensor signal. The adjustment may be made on the basis of a calculation, or a calibration. The calculation or calibration may be stored in a memory.

The controller may control operation of the UV source.

For example, the controller may switch the UV source repetitively on and off. It may also regulate the duration of each on period and the time between each on period.

The controller may switch the UV source from a state of continuous illumination to a state of intermittent operation. The controller may switch the way the UV source operates dependent on the measurement signal, that is dependent on the measured concentration of analyte in the sample gas. For example, the controller may operate the UV source in a state of continuous illumination so long as the measured concentration of analyte in the sample gas remains below a first concentration threshold. The controller may then change the operation of the UV source to a state of intermittent illumination when the measured concentration of analyte in the sample gas exceeds the first concentration threshold. The controller may adjust the frequency of intermittent operation dependent on the measured concentration of analyte. For example, the UV source may be illuminated intermittently at a first frequency below a second concentration threshold, and then illuminated intermittently at a second frequency above the second concentration threshold. The second frequency may be lower than the first frequency. The second frequency may be higher than the first frequency.

During illumination of the UV source the controller may regulate the power to the UV source. The controller may regulate the power to the UV source dependent on a measured light output of the source. The light output of the source may be measured by a photosensor. The photosensor may measure ultraviolet radiation emitted into the ionization chamber of the PID. Contamination may build up on the UV window, for example from the deposition of VOC molecules on the UV window. As this contamination absorbs radiation, contamination on the window surface results in a reduction in the intensity of radiation emitted into the ionization chamber. The power to the UV source may be increased to compensate for this reduction in ionizing radiation. Alternatively, the measured reduction in intensity could be used to compensate the reduced measured signal before output as either analogue or digital. The controller may also regulate the power to the UV source to compensate for loss of emission due to aging of the lamp. The ionizing (UV) radiation output of the UV source may be measured indirectly for this purpose. For example, a photosensor detecting light emitted through a side wall of the source bulb may be used to monitor changes due to aging of the source.

In a further aspect the invention discloses a method of operating a photo-ionization detector (PID), wherein the PID comprises a UV source and a first electrode for generating a measurement signal, in which method control of the UV source depends on the measurement signal generated at the first electrode.

A further aspect the invention relates to a method of operating a photo-ionization detector (PID), wherein a controller varies the frequency of the current driving the UV source of the PID to facilitate finding the optimum frequency. The optimum frequency is typically the resonant frequency of the electrical circuit which includes the UV source. The optimum or resonant frequency can vary with time, for example due to aging of the UV source, and with parameters such as temperature and humidity. The controller may monitor the current in the electrical circuit of which the UV source is part while varying the frequency of the driving current thereby determine the resonant or optimum frequency. In a resonance seek mode, the controller may perform a wide range frequency sweep to determine the resonant frequency. This may be done during a strike phase. In a resonance track mode, the controller may perform a frequency sweep over a narrow range around a previously determined resonant frequency. A resonance track mode frequency sweep may be carried out during an illumination phase of the UV source, while the UV source is emitting UV radiation. In this way changes to the physical system resonant frequency can be detected and adjusted for. Following a resonance track mode sweep, the controller may drive the UV source at the resonant frequency established during the resonance track mode sweep last performed.

In a further aspect the present disclosure extends to a method of maintenance of a photo-ionization detector (PID), the photo-ionization detector comprising a main body, in which method a replaceable electrode stack module comprising a first electrode is combined with the main body, so that the first electrode can be used to detect gaseous analyte. The replaceable electrode stack may additionally comprise an second electrode of the PID and where appropriate a guard electrode. Further elements of the PID may form part of the replaceable electrode stack. For example, the bulb of the UV source may form part of the replaceable electrode stack. A memory circuit may also form part of the replaceable electrode stack.

The electrodes of the PID are subject to contamination from the sample gas, and in particular from constituents of the sample gas such a VOCs. This can result in the electrodes having a useful lifetime, after which they require replacement. Having the electrodes as part of a replaceable electrode stack facilitates their replacement when this is required. Maintaining the PID by replacing the electrode stack is a cost-effective way of extending the lifetime of the PID.

Similarly, having the UV bulb, and possibly the electrodes of the UV source, as part of a replaceable electrode stack module enables a depleted UV source to be replaced. The UV bulb may be a replaceable part of the replaceable electrode stack. The UV bulb and associated UV source electrodes may be replaceable parts of the replaceable electrode stack.

Where the replaceable electrode stack additionally comprises a memory circuit, this may log the operational time of the electrodes of the replaceable stack, i.e. the amount of time during which the electrodes have been in use in the PID. The memory circuit may also log the total illumination time of the UV source. Further parameters relating to the operational history of the electrodes and/or the UV source may be recorded in the memory circuit. In this way the memory chip may be configured to output a signal indicating when the electrode stack requires replacement. The memory circuit may also record the integral of the measured gas exposure. This is useful as the stack lifetime is gas concentration dependent. For example, the stack lifetime will be significantly lowered when subject to low gas concentration, in contrast to high concentration.

The replaceable electrode stack may also comprise a sensor for measuring a condition of the sample gas, such as a humidity and/or temperature sensor. The accuracy of the humidity sensor, for example, may reduce over time due to fouling. Thus to maintain correct readings, it is advantageous for the humidity sensor to be replaced at the same time as the electrode stack. The humidity sensor is advantageously replaced at the same time as the electrode stack if it is part of the electrode stack.

The controller may be configured to measure the drive current passing through the UV source as the frequency of the current is varied.

In a further aspect, the present disclosure relates to a method of operating a photoionization detector (PID) comprising a UV source, an second electrode, a first electrode, and a guard electrode, wherein: a first potential is applied to the guard electrode; while the guard electrode is maintained at said first potential, a first measurement is taken from the first electrode and a first background measurement is taken from the guard electrode; a second potential is applied to the guard electrode; while the guard electrode is maintained at said second potential, a second measurement is taken from the first electrode and a second background measurement is taken from the guard electrode; a corrected measurement is produced from said first measurement, said second measurement, said first background measurement and said second background measurement. Said first potential may be positive and said second potential may be negative. Alternatively, said first potential may be negative and said second potential may be positive. As a further alternative, said first potential and said second potential may both be positive or negative, but may be of different magnitudes.

Typically, each of the first measurement, the second measurement, the first background measurement and the second background measurement is a current measurement.

The corrected measurement may be the current generated at the first electrode due to positive VOC ions landing on the first electrode. Typically each of the first measurement and the second measurements is a total current generated at the first electrode. This total current typically includes a contribution from positive VOC ions landing on the first electrode and from electrons which are photoemitted from the first electrode.

### Description of the Drawings

An example embodiment of the present invention will now be illustrated with reference to the following Figures in which:
Figure 1 shows a cross sectional cut-away of the PID;
Figure 2 shows the PID in two partially stripped-down configurations. The stripped-down PID of Figure 2(a) includes the electrode stack; the electrode stack is not present in the stripped-down PID of Figure (b);
Figure 3 shows a schematic cross-sectional diagram of a replaceable electrode stack including a UV source bulb and a temperature and humidity sensor;
Figure 4 shows a replaceable electrode stack module located in a PID and connections between the electrode stack module and the remaining parts of the PID;
Figure 5(a) illustrates the operational principal of the PID with a positive potential applied to the guard electrode; Figure 5(b) illustrates the effect of polarising the guard electrode with a negative potential; Figure 5(c) illustrates the effect of changing the polarity of each of the second electrode, first electrode, and guard electrodes;
Figure 6 shows a schematic diagram of the PID arrangement;
Figure 7 shows a block diagram of the PID sensor electronics;
Figure 8 shows a simplified schematic diagram of the PID sensor electronics;
Figure 9 details the circuit powering the UV source and the H-bridge used to drive the UV-source circuit;
Figure 10 shows an example of signal logic applied to the H-bridge and the signal which is used to power the UV source and that which is used to bias the second electrode which result;
Figure 11 shows a further example of signal logic applied to the H-bridge and the signal which is used to power the UV source and that which is used to bias the second electrode which result;
Figure 12(a) illustrates the resonance seek mode; (b) the current response from the UV source circuit to the resonance seek frequency scan; (c) the resonance track mode; and (d) the corresponding current response to the resonance track frequency scan.
Figure 13 is an illustration of the resonant frequency tracking method;
Figure 14 illustrates how the UV source may be switched between continuous operation and intermittent operation dependent on the VOC concentration;

### Detailed Description of an Example Embodiment

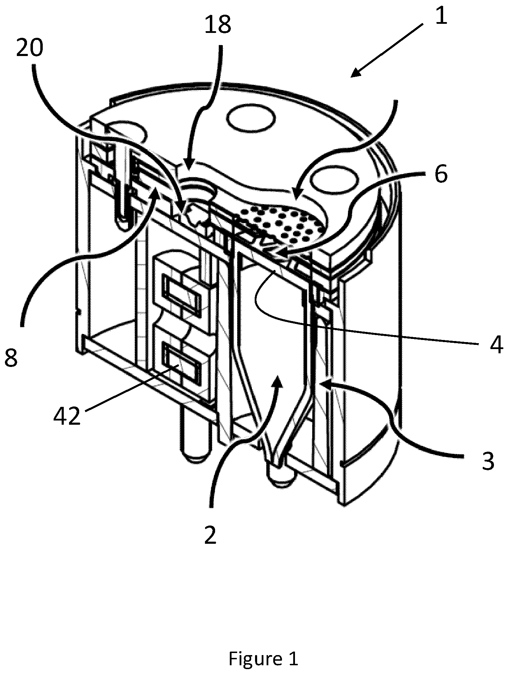

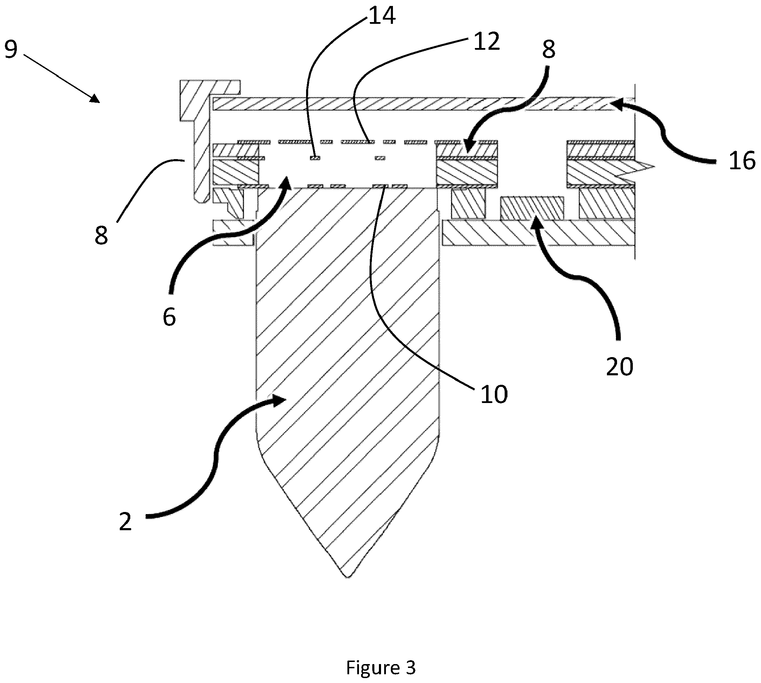

Figure 1 shows a schematic cross section of an embodiment of the photo-ionization detector (PID) 1. UV source 2 is formed of a closed bulb which contains a noble gas such a krypton. The choice of gas within the bulb determines the wavelength of UV radiation produced by the UV source and will be made dependent on which VOCs are of principal interest. The PID comprises a housing. UV source 2 sits in lamp cavity 3, which forms part of the PID housing. Electrical contacts (not shown) are positioned around the bulb to excite the gas and produce UV excitation. The UV radiation emits through a UV window 4 at the flat end of the bulb, passing into and through the ionization chamber 6 sitting immediately above the bulb. Electrode stack 8 is placed at the exit of the UV source. In the present embodiment electrode stack 8 comprises second electrode 10, first electrode 12, and guard electrode 14. Second electrode 10 is positioned closest to the exit of the UV window 4, and first electrode 12 is placed furthest away. First electrode 12 is the first electrode. Guard electrode 14 is placed in-between second electrode 10 and first electrode 12. Dust filter 16 is positioned at the gas entrance to the PID (see Figure 3).

Sensor chamber 18 is fluidically connected to ionization chamber 6 within the sensor. Positioned in sensor chamber 18 is temperature and humidity sensor 20, which measures the temperature and the humidity of any sample gas which is being sensed by the PID.

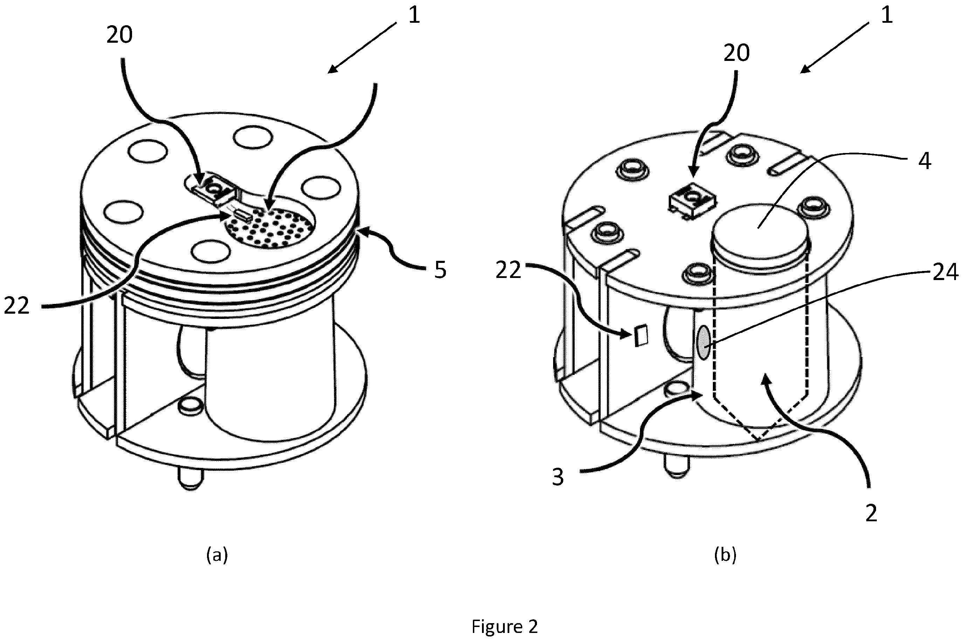

Figure 2(a) shows an embodiment of the PID stripped of the outer wall of the housing and Figure 2(b) shows an embodiment of the PID with the electrodes of the electrode stack also removed. The embodiments of Figures 2(a) and 2(b) each include a photosensor 22. In Figure 2(a) photosensor 22 is placed so that it picks up radiation emitted through window 4 of the UV source. In the embodiment of Figure 2(b) photosensor 22 is placed within the sensor housing in such a position that it picks up radiation emitted through the bulb wall of UV source 2 which passes through window 24 in lamp cavity 3. In either configuration, photosensor 22 monitors the UV output of source 2, either directly by measuring the ionizing radiation used by the PID (Figure 2(a)), or indirectly, by measuring additional radiation produced by the source (Figure 2(b)).

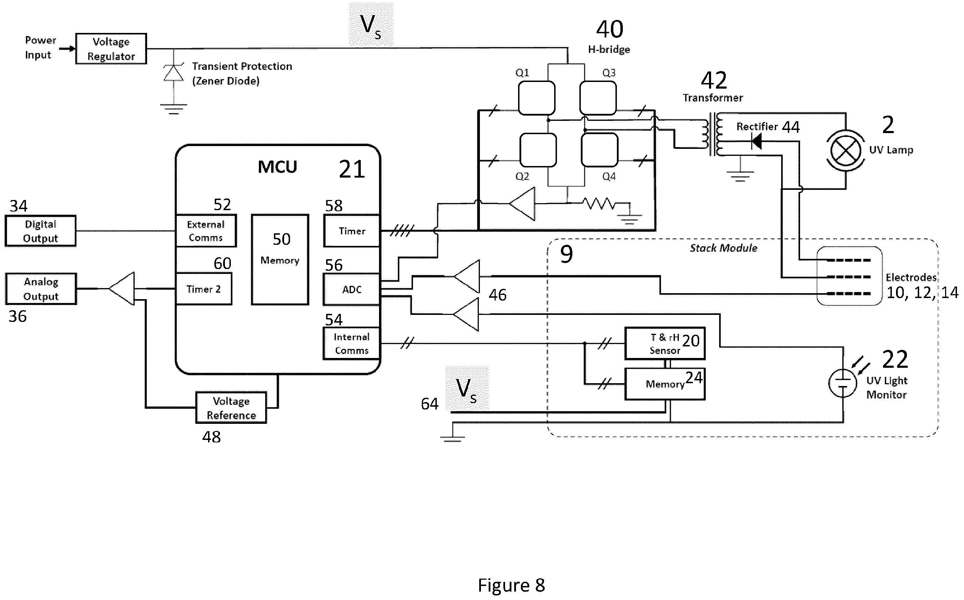

In one embodiment, the electrode stack takes the form of a replaceable module 9 (Figure 3). The replaceable electrode stack module 9 comprises all the electrodes of the PID held in a mounting block. Other features of the PID which may form part of the replaceable electrode stack 9 include the UV source 2 and the temperature and humidity sensor 20. Figure 3 shows an embodiment of a replaceable electrode stack 9 which comprises second electrode 10, first electrode 12, guard electrode 14, UV source 2 and temperature and humidity sensor 20. The replaceable electrode stack 9 may also comprise a memory 24, such as a solid-state memory chip (see Figure 8).

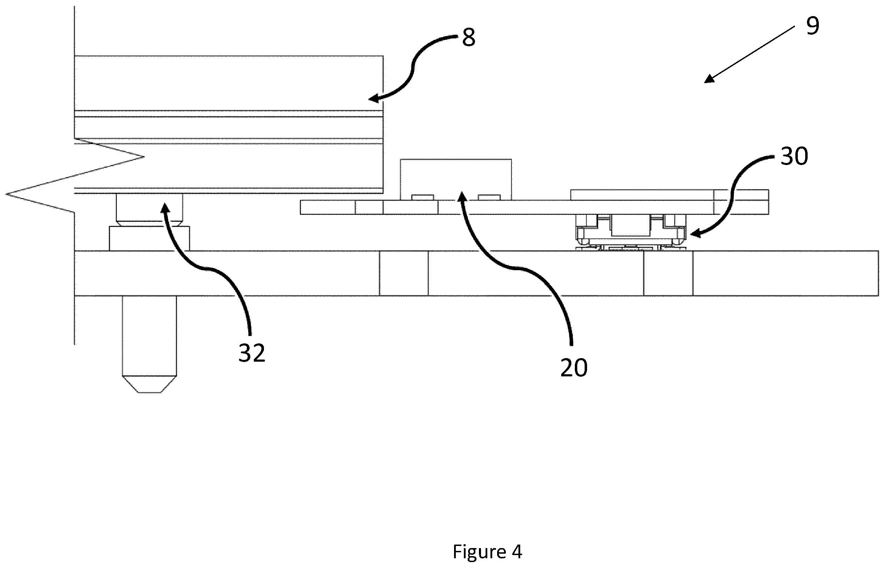

Figure 4 shows an example of how electrical connections are made from the main PID body to the replaceable electrode stack 9. The stack of electrodes 8 is schematically shown, as is the temperature and humidity sensor 20. Main stack connection 30 provides a means for making electrical connections between the elements of the replaceable electrode stack 9 and the rest of the PID, as does auxiliary stack connection 32. Suitable provisions are made for both analogue signals and digital signals to be conveyed to and from elements of the electrode stack. Such signals include the voltages which are supplied to the electrodes of the electrode stack for the PID to operate; current signals, for example from the first electrode; electrical signals to and from the temperature and humidity sensor, for example to power the sensor or to take readings from the sensor; and electrical signals to any other electrical or electronic element which may form part of the electrode stack module.

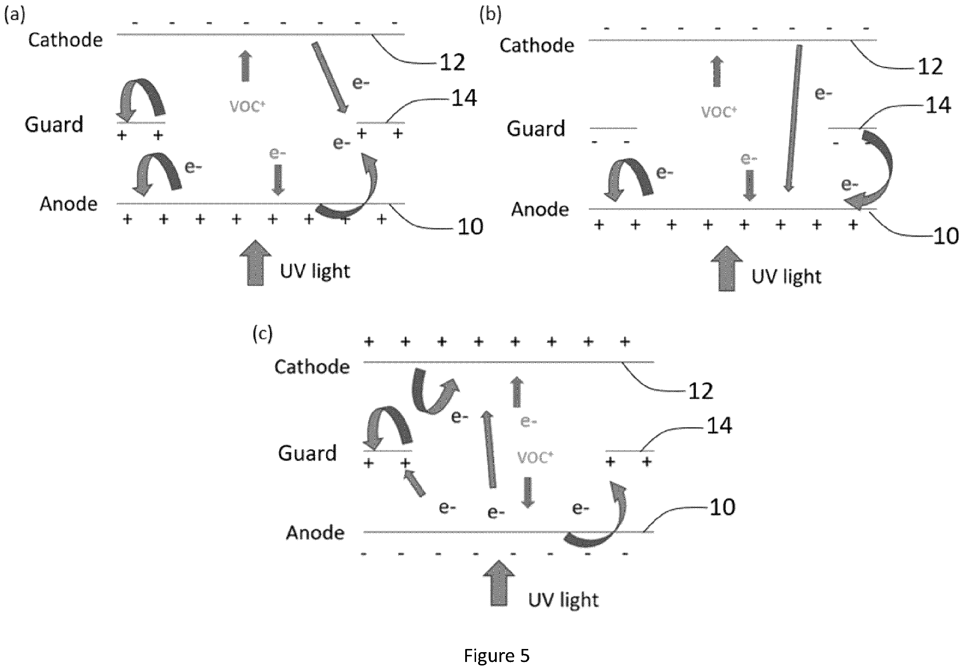

The basic operational principle of a PID is illustrated in Figure 5(a). In the present device, the sample gas enters the PID ionization chamber by diffusion. Although it is also known to draw sample gas into a PID using a pump, the present inventors have found that this is not necessary in the current device and that gas diffusion is a sufficiently efficient way to supply gas to the device to ensure sampling of the environment of the sensor. The chemical species of interest (gaseous analyte) within the sample gas are generally volatile organic compounds (VOCs). VOC molecules are ionized in the ionization chamber by the UV radiation from the UV source 2. The electric field present in the ionization chamber produced by potentials applied to the electrodes of the electrode stack causes the ionised VOCs to drift. The positively charged VOC ions drift towards the negatively charged first electrode, here measurement cathode 10, where a signal is produced when they land on the first electrode surface. Photoelectrons are produced by UV light impinging on surfaces within the ionisation chamber and are mopped up either by positively charged second electrode, here anode 10, or by a positively charged guard electrode 14.

In an alternative voltage arrangement (Figure 5(b)), the guard electrode 14 may be connected to a negative potential, whereby photoelectrons will be repelled away from the guard electrode 14 towards the second electrode (or anode) 10 where they will be absorbed.

Photoemission from the first electrode (or cathode) 12 causes a current signal which interferes with the measurement signal arising due to positive VOC ions landing on the first electrode. The current due to ionised gas molecules may be deconvoluted from the current due to photoemission from the first electrode by changing between a configuration in which the guard electrode 14 is held at a positive potential (Figure 5(a)), and a configuration in which the guard electrode 14 is held at a negative potential (Figure 5(b)).

When the guard electrode 14 is held at a positive potential, photoelectrons from the surface of the first electrode 12 are attracted to the guard electrode 14 where they may be neutralised, resulting in a current from the guard electrode 14. When the guard electrode 14 is held at a negative potential, the same photoelectrons are repelled from the guard electrode 14. The change in current from the guard electrode 14 between these two configurations is thus a function of the contribution to the signal from the first electrode 12 from photoemitted electrons. An increase in measurement accuracy may thereby be achieved by isolating the measurement signal produced by the VOC ions alone.

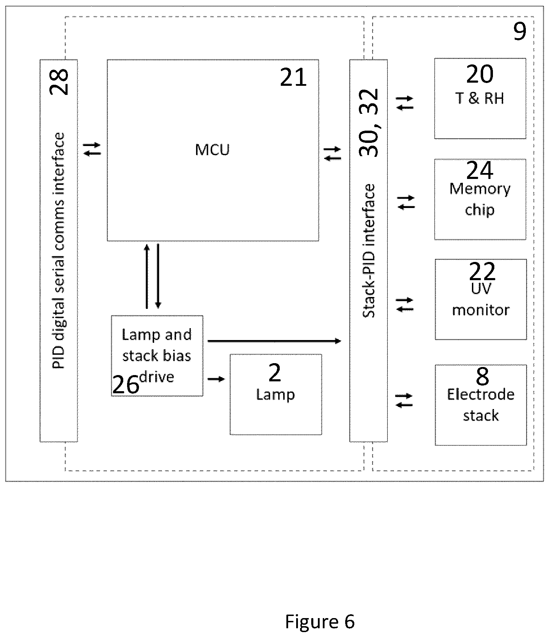

In a preferred embodiment the PID comprises a microcontroller unit (MCU) 21, for example a solid-state microchip, which controls the device. Figure 6 shows a schematic diagram of the PID sensor electronics including the microcontroller unit 21. This embodiment includes an interface 30, 32 between the PID and the electrode stack module 9 comprising the electrodes (second electrode, first electrode and guard electrode), the UV monitor 22, a memory chip 24, and the temperature and humidity sensor 20. A digital serial communication interface 28 is also included enabling an external controller to be connected to the microcontroller unit 21. In this embodiment, the microcontroller 21 also controls the function of the UV source 2. A bias drive unit 26 provides a common interface between the microcontroller 21 and the UV source 2 and the electrode stack module 9.

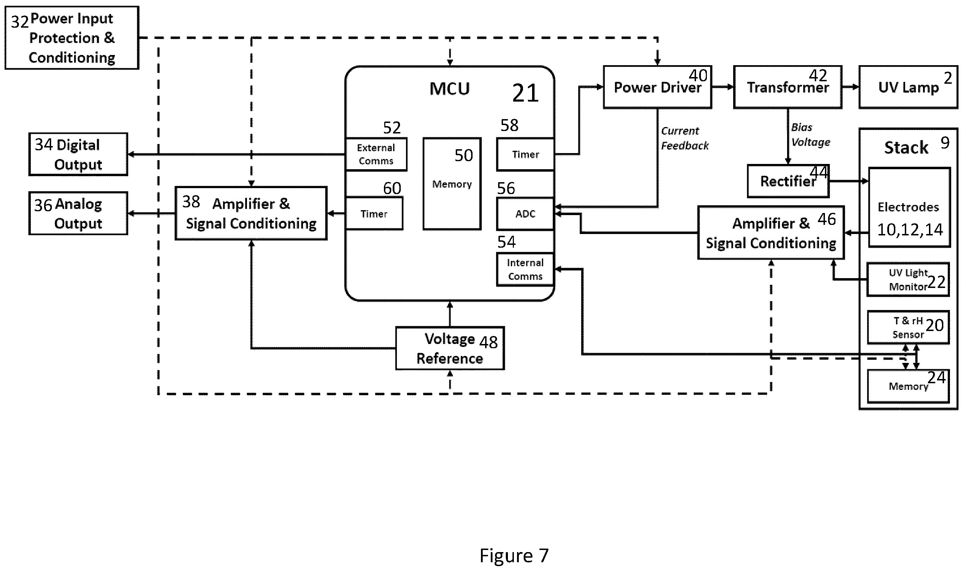

A further schematic block diagram illustration of connections between the microcontroller 21, elements of the electrode stack module 9, and external controls for an exemplary embodiment is shown in Figure 7, with a simplified schematic diagram in Figure 8. Power driver 40 supplies power to UV lamp 2 and, through rectifier 44, supplies voltages to the PID electrodes of the electrode stack module 9. Other elements of the electrode stack module 9 which require power, such as UV light monitor 22, temperature/humidity sensor 20 and memory unit 24 are powered by a separate source 64 (see Figure 8).

The frequency of the signal which drives the UV lamp is determined by a timer, for example a counter timer circuit, 58 in MCU 21 which supplies timing signals to power driver 40. In this embodiment, power driver 40 takes the form of an H-Bridge, as shown in Figure 8. The AC signal from power driver 40 is passed to the primary coil of transformer 42. Transformer 42 also comprises a secondary coil which produces a transformed voltage suited for powering the UV lamp 2. In the embodiment illustrated in Figures 7 and 8 a bias voltage is additionally extracted from the transformer 42. The bias voltage passes though rectifier 44 before being used to bias first electrode 10 of the electrode stack.

Microcontroller unit 21 comprises analogue to digital convertor 56, into which it receives an analogue measurement signal from first electrode 12, being the measurement signal from the electrode stack, an analogue measurement signal from UV light monitor 22, representing the amount of light monitored by the monitor, and a current feedback signal, monitoring the current passing through H-Bridge 40. Digital signals are produced by the ADC for further use by the MCU.

Solid state memory 24 and temperature and humidity sensor 20 are present in the electrode stack module and are connected to the MCU 21. In this way memory 24 can be used by the MCU 21 to record a history of readings from the temperature and humidity sensor. Memory 24 can also record the total amount of time that the temperature and humidity sensor 20 of the module 9 has been operating. A key benefit of the memory is to store data which is specific to the specific stack, such as calibration data for the stack. Calibration data for the stack may be programmed or written to the memory during final test of the stack in manufacture.

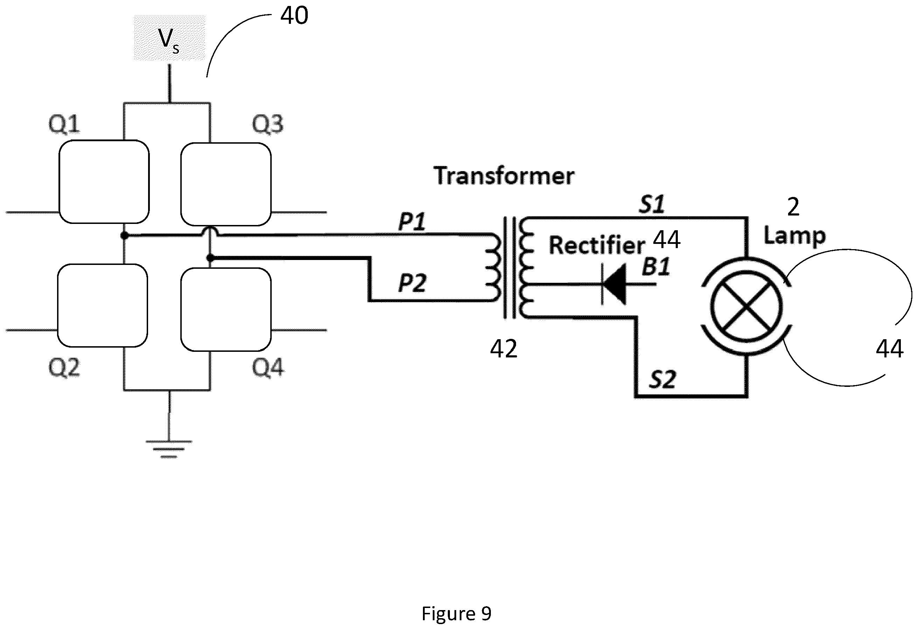

Figure 9 shows an arrangement for providing power through power driver 40 and transformer 42 to UV source 2. In this arrangement, a bias voltage is also produced by tapping off and rectifying a voltage output from the secondary coil of transformer 42. The bias voltage is used to bias one or more of the plurality of electrodes.

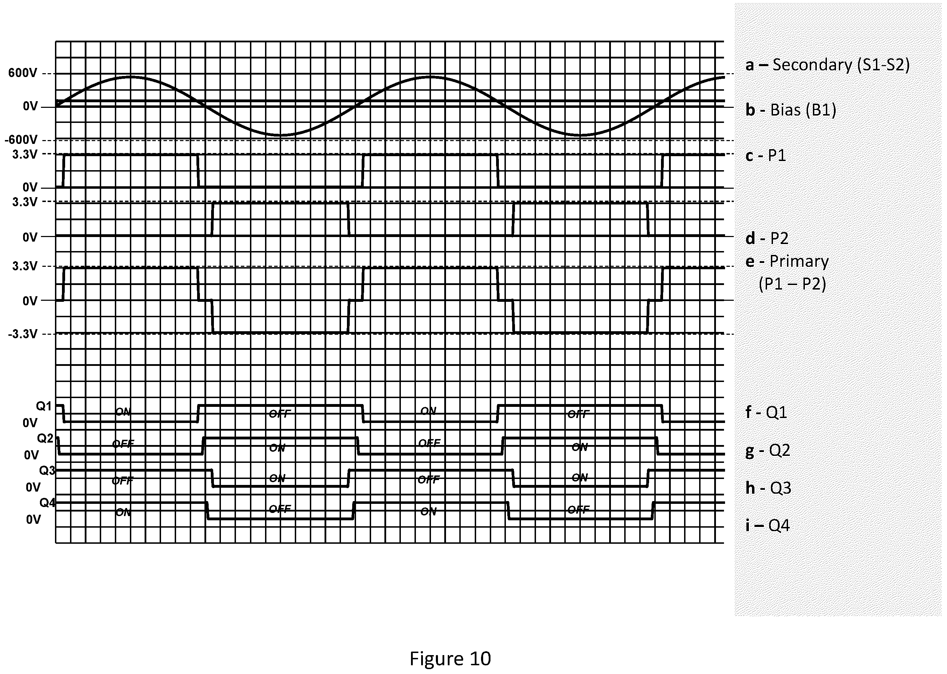

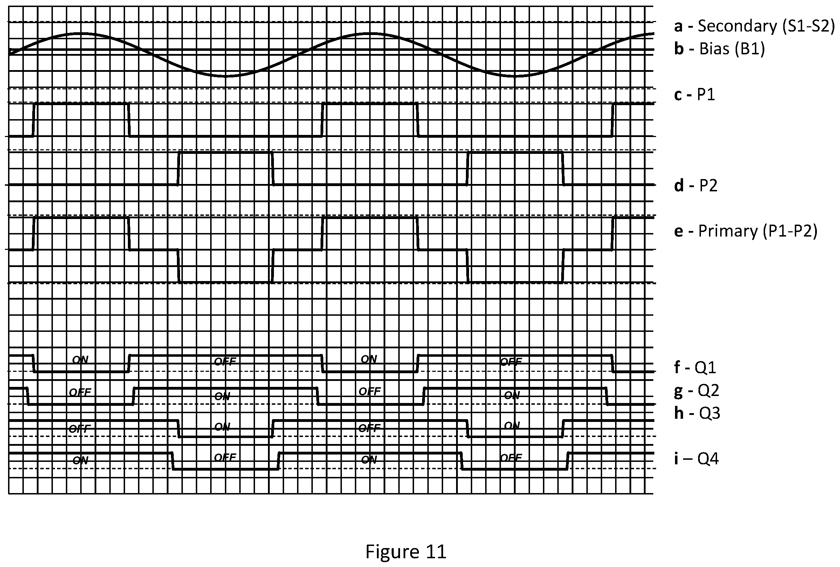

The UV source is powered by supplying it with an AC signal, typically in the radio frequency range. H-bridge circuit 40 is used in combination with transformer 42 to convert a DC signal to the AC signal suitable to power the source. By regulating the timing of the logic signals sent to transistors Q1, Q2, Q3 and Q4 the power supplied to the UV source 2 is controlled. Figures 10 and 11 show how different duty cycles can be exploited to vary the amplitude of the voltage supplied to the UV source 2. Figure 10 shows how, using a 200 turn ratio transformer 42 with a 6.6 V peak-to-peak input of 94% duty cycle, a peak-to-peak output voltage of ± 600V is achieved. Reducing the duty cycle to 67% halves the peak-to-peak voltage to ± 300V, as shown in Figure 11. The frequency of the signal sent to the UV source 2 also depends on the timing of the signals sent to transistors Q1-Q4.

UV source 2 and electrodes 44 powering it form an element in a resonating electrical circuit. The properties of this element may vary as the UV source 2 ages, which may lead to the UV source 2 being driven in a sub-optimal manner, even including an inability to ignite the UV source 2. Changes in temperature of the transformer may also result in changes to the optimal drive frequency of the UV source.

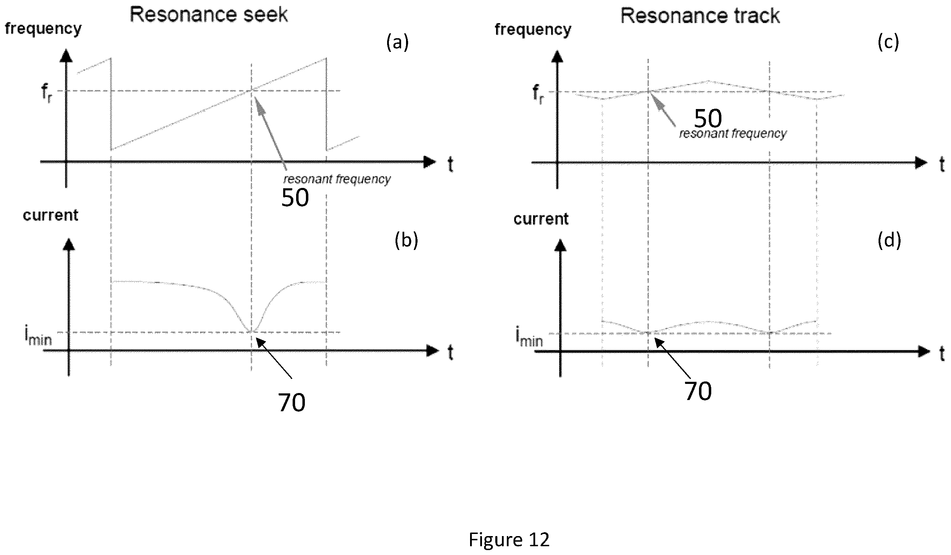

The present invention addresses this inherent loss of efficiency of the UV source 2 by either seeking or tracking (as appropriate) the resonant frequency 50 of the UV source circuit 2. The resonance seek mode implements a sweep of frequencies (Figure 12(a)) to identify the resonant frequency 50 of the circuit including the UV source. This sweep may take place during a strike phase to illuminate the UV source for the first time. The resonance seek mode may also be utilised following a particular event, such as an excessive temperature step change, an excessive current step change, a significant input power supply voltage change, or a failed resonance track mode frequency sweep, to name a few examples. The resonance seek mode renders the presently stored resonance frequency as potentially invalid. A linear frequency sweep is performed, either between two fixed end points, or from a fixed start frequency and utilising a predetermined frequency range. The lamp driver current is continuously monitored during the frequency sweep. For example, this may be done by monitoring the current feedback from power driver 40 in Figure 7. The resonance point 50 is identified as the point with the minimum measured current 70 within the expected operational band (see Figure 12(a)(b)). For a resonance around or expected to be around 120 kHz, the resonance sweep may start at 80 kHz and sweep in steps of 1 kHz for example to 160 kHz, say.

In contrast, the resonance track mode is a narrow frequency sweep in the vicinity of the known resonance - see Figure 12(c). The resonance track mode may be activated periodically or triggered by an event such as may trigger the resonance seek mode. In the resonance track mode, the frequency driving the UV source is deemed to be close to the actual physical resonance frequency. A linear frequency sweep is made over a predetermined frequency range, where the central frequency is the drive frequency presently being used. The range of the frequency sweep in the resonance track mode is significantly smaller than that of the resonance seek mode. In the resonance seek mode a sweep may be performed from 80 kHz to 140 kHz to locate the resonance. A step size of 2kHz may be used. In the resonance track mode the linear sweep may be carried out with a step size of 100 Hz or 200 Hz, possibly about 5 kHz above and below resonance. As shown in Figure 12(d), the minimum point in the current response 70 enables the resonance frequency 50 to be identified. The resonance track mode may be carried out periodically or even dynamically during measurement in order to detect any changes in the resonant frequency and to adjust the drive frequency accordingly to keep exciting the UV source at the resonant condition.

Each of the resonance seek mode and the resonance track mode may be run in an adaptive resonance track mode. In the adapted resonance track mode values of previously identified resonant frequencies are stored in a memory, such as memory 24 of a particular stack module 9, or memory 50 of the microcontroller unit of the PID, along with measured environmental conditions, such as temperature and/or humidity, and specific device parameters, such as bias voltages, device age, device history. A system of machine learning can then be used to enable faster identification of the resonant frequency appropriate to the present condition of the PID.

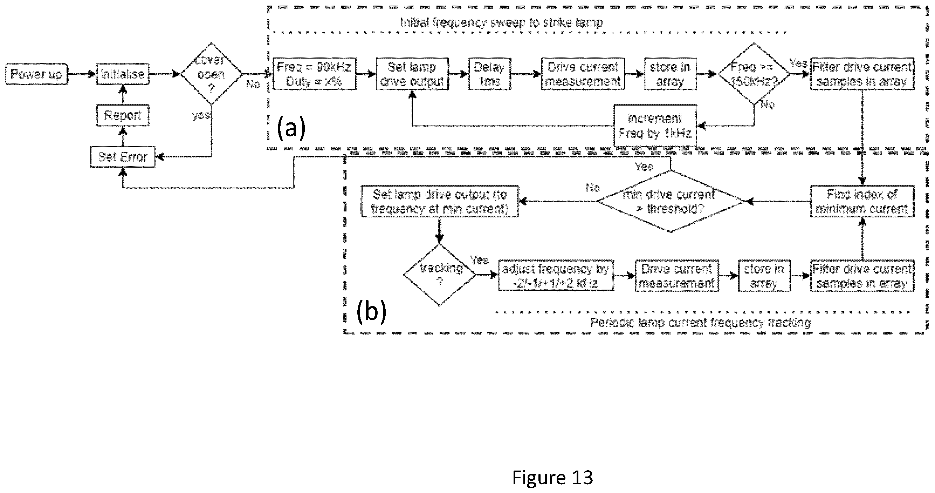

Figure 13 shows an algorithm for operating the UV source 2, which incorporates (a) a strike phase and (b) a frequency tracking phase, which periodically tracks the frequency of the signal powering the UV source. If the minimum drive current at resonance is greater than a predetermined threshold, an error is output.

As part of the strike phase, an initial frequency sweep (a) to determine the resonant frequency is performed. This is called a resonance seek mode. In a resonance track mode, the controller periodically performs a resonance track mode sweep (b) over a narrow range around a previously determined resonant frequency. A resonance track mode sweep may be carried out during an illumination phase of the UV source, while the UV source is emitting UV radiation. In this way changes to the resonant frequency of the physical system can be detected and adjusted for in a dynamic manner, i.e. as they occur over time and as the PID is operating. Following a resonance track mode sweep, the controller may proceed to drive the UV source at the resonant frequency established during the resonance track mode sweep last performed. The controller may drive the UV source at the newly established resonant frequency until such time as the next resonance track mode sweep is deemed necessary. A resonance track mode sweep may be carried out, for example, at time intervals which may be fixed, or may be dependent on the cumulative operational time of the PID, or which may depend on the output of the UV monitor, or another monitor of the output of the UV source.

The radiation emitted into the ionization chamber 6 by the UV source 2 is also subject to the build-up of contamination on the surface of the UV window 4. This contamination occurs through the presence of the very VOC molecules the PID is designed to detect. The present invention envisages monitoring the radiation output of the UV source 2 and using this in a feedback loop to adjust the power supplied to illuminate the source 2. As the radiation intensity into the ionization chamber drops off due to the presence of contamination on the UV window 4, a feedback loop increases the power supplied to the UV source 2 to increase its output. In this way a constant radiation intensity can be maintained, leading to more accurate measurements. The radiation output of the UV source 2 can be monitored by using photosensor 22 in the ionization chamber 6. The photosensor may be one dedicated to this purpose. It is also possible to use a current from the guard electrode 14 or from the second electrode 10 as a radiation monitor for this purpose.

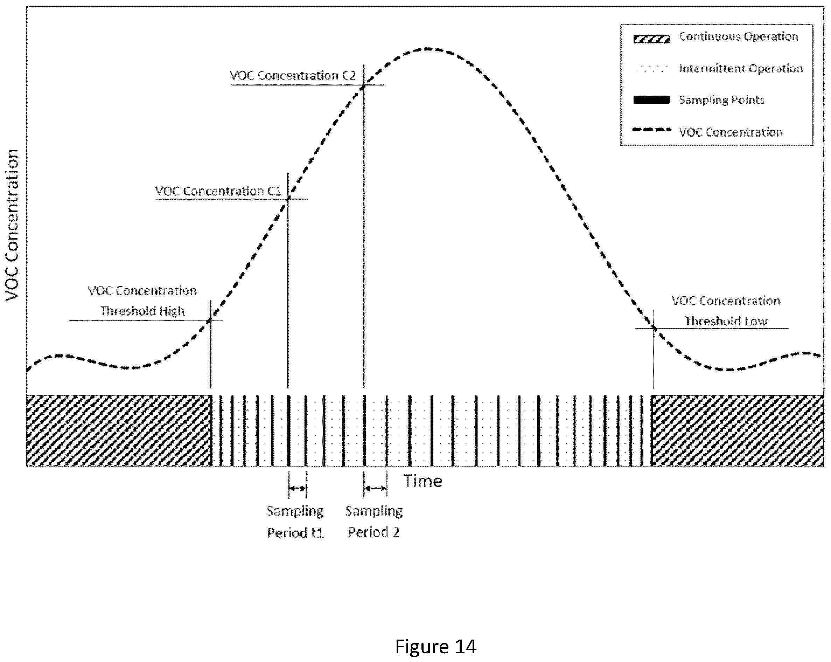

During operation of the PID the UV source may be illuminated continuously, that is so that it emits UV radiation continuously, or it may be illuminated intermittently, that is emitting UV radiation during short periods of time, between which no radiation is emitted by the UV source. Figure 14 illustrates a manner of operating the PID in which the UV source is illuminated continuously so long as the measured VOC concentration stays below a first predetermined threshold. Two mechanisms of operation are illustrated in this figure. The first mechanism is a switch from continuous operation to intermittent mode. When the VOC concentration exceeds the first predetermined threshold, the UV source operation is changed to one of intermittent illumination at a set sampling period t1. The second mechanism of operation is a switch within the intermittent mode. If the measured VOC concentration continues to increase beyond a second predetermined concentration C1, the time between radiation bursts from the UV source may be increased so that a second sampling period t2 is achieved. In this way, operation of the UV source may be matched to the measured VOC concentration and energy may be saved by not powering the UV source continuously.

The following numbered clauses form part of the present disclosure.
1. A photo-ionization detector (PID) comprising: a UV source; an ionization chamber for receiving sample gas; a plurality of electrodes, including a first electrode, for detecting gaseous analyte ionized in the ionization chamber; a controller; and at least one sensor in electronic communication with the controller for measuring a condition of the sample gas.
2. A PID according to clause 1, wherein the plurality of electrodes is part of a replaceable electrode stack module.
3. A PID according to clause 2, wherein the at least one sensor comprises a humidity and/or a temperature sensor, and the replaceable electrode stack module comprises the humidity and/or temperature sensor.
4. A PID according to clause 3, wherein the humidity and/or temperature sensor is positioned in a chamber which is separate to but in fluid communication with the ionization chamber.
5. A PID according to any one of clauses 2 to 4, wherein the electrode stack module comprises a memory.
6. A PID according to any one of clauses 2 to 5, wherein the electrode stack module comprises the UV source.
7. A PID according to any one of clauses 2 to 6, wherein the electrode stack module comprises a UV monitor.
8. A PID according to any one preceding clause, wherein the controller is a microprocessor or microcontroller.
9. A PID according to clause 8, comprising a plurality of electrical connections for outputting an analogue measurement signal and a plurality of separate electrical connections for outputting a digital measurement signal.
10. A PID according to any one preceding clause, wherein operation of the PID is controlled by the controller, responsive to data stored in the memory of the PID or, where applicable, the memory of the electrode stack module.
11. A PID according to any one preceding clause, wherein operation of the PID is controlled by the controller responsive to sensor data from the at least one sensor.
12. A PID according to clause 10 or clause 11, wherein the operation which is controlled may comprise operation of the UV source.
13. A PID according to clause 12, wherein the controller is configured to switch the UV source repetitively on and off and optionally to regulate the duration of each on period and the time between each on period.
14. A PID according to clause 12 or clause 13, wherein the proportion of time for which the UV source is on is reduced by the controller to reduce power consumption or to extend source lifetime.
15. A PID according to any one of clauses 12 to 14, wherein the proportion of time for which the UV source is on is reduced by the controller responsive to measurements of VOC concentration.
16. A PID according to any one preceding clause, wherein the controller is configured to regulate the power to the UV source, when it is on, to switch it between a plurality of different power levels in a cycle.
17. A PID according to clause 16, wherein the plurality of power levels comprises a strike phase, followed by at least one illumination phase.
18. A PID according to clause 16, wherein during the strike phase, the controller is configured to vary the frequency of the current driving the UV source to facilitate finding the optimum frequency (which can vary with time and parameters such as temperature and humidity).
19. A method of controlling a photo-ionization detector (PID), in which method a sample gas enters (typically diffuses) into an ionization chamber of the PID;
   said sample gas in the ionization chamber is irradiated by UV radiation from a UV source;
   a first electrode generates a first signal indicative of detected gaseous analyte;
   at least one sensor measures at least one condition of the sample gas and generates at least one sensor signal;
   a controller receives the first signal and the at least one sensor signal;
   the controller outputs a measurement signal determined from the first signal and compensated taking into account the at least one sensor signal.
20. A method according to clause 19, wherein the at least one sensor comprises a humidity and/or a temperature sensor.
21. A method according to either of clause 19 and clause 20, wherein the controller controls operation of the UV source.
22. A method according to clause 21 wherein the controller switches the UV source repetitively on and off and optionally regulates the duration of each on period and the time between each on period.
23. A method according to any one of clauses 19-22, wherein during illumination of the UV source the controller regulates the power to the UV source.
24. A method of operating a photo-ionization detector (PID), wherein the PID comprises a UV source and a first electrode for generating a measurement signal, in which method control of the UV source depends on the measurement signal generated at the first electrode.
25. A method of operating a photo-ionization detector (PID), wherein a controller varies the frequency of the current driving the UV source of the PID to facilitate finding the optimum frequency (which can vary with time and parameters such as temperature and humidity).
26. A method of maintenance of a photo-ionization detector (PID), the photo-ionization detector comprising a main body, in which method a replaceable electrode stack module comprising a first electrode is combined with the main body, so that the first electrode can be used to detect gaseous analyte.
27. A method of maintenance of a photo-ionization detector (PID) according to clause 26, wherein the replaceable electrode stack module comprises a UV source.
28. A method of maintenance of a photo-ionization detector (PID) according to either of clause 26 and clause 27, wherein the replaceable electrode stack module comprises a solid state memory, which is used for calibration.
29. A method of operating a photoionization detector (PID) comprising a UV source, an second electrode, a first electrode, and a guard electrode, wherein:
   a positive potential is applied to the guard electrode
   while the guard electrode is maintained at said positive potential, a first measurement is taken from the first electrode and a first background measurement is taken from the guard electrode;
   a negative potential is applied to the guard electrode;
   while the guard electrode is maintained at said negative potential, a second measurement is taken from the first electrode and a second background measurement is taken from the guard electrode;
   a corrected measurement is produced from said first measurement, said second measurement, said first background measurement and said second background measurement.

## Claims

1. A photo-ionization detector (PID) comprising: a UV source; an ionization chamber for receiving sample gas; a plurality of electrodes, including a first electrode, for detecting gaseous analyte ionized in the ionization chamber; a controller; and at least one sensor in electronic communication with the controller for measuring a condition of the sample gas.

2. A PID according to claim 1, wherein the plurality of electrodes is part of a replaceable electrode stack module, wherein optionally the at least one sensor comprises a humidity and/or a temperature sensor, and the replaceable electrode stack module comprises the humidity and/or temperature sensor, wherein as a further option the humidity and/or temperature sensor is positioned in a chamber which is separate to but in fluid communication with the ionization chamber.

3. A PID according to claim 2, wherein the electrode stack module comprises a memory, wherein optionally the electrode stack module comprises the UV source, wherein optionally the electrode stack module comprises a UV monitor.

4. A PID according to any one preceding claim, wherein the controller is a microprocessor or microcontroller, the PID optionally comprising a plurality of electrical connections for outputting an analogue measurement signal and a plurality of separate electrical connections for outputting a digital measurement signal.

5. A PID according to any one preceding claim, wherein operation of the PID is controlled by the controller, responsive to data stored in the memory of the PID or, where applicable, the memory of the electrode stack module.

6. A PID according to any one preceding claim, wherein operation of the PID is controlled by the controller responsive to sensor data from the at least one sensor.

7. A PID according to claim 5 or claim 6, wherein the operation which is controlled may comprise operation of the UV source, wherein optionally the controller is configured to switch the UV source repetitively on and off and optionally to regulate the duration of each on period and the time between each on period, wherein optionally the proportion of time for which the UV source is on is reduced by the controller to reduce power consumption or to extend source lifetime, wherein optionally the proportion of time for which the UV source is on is reduced by the controller responsive to measurements of VOC concentration.

8. A PID according to any one preceding claim, wherein the controller is configured to regulate the power to the UV source, when it is on, to switch it between a plurality of different power levels in a cycle, wherein optionally the plurality of power levels comprises a strike phase, followed by at least one illumination phase, wherein optionally during the strike phase, the controller is configured to vary the frequency of the current driving the UV source to facilitate finding the optimum frequency (which can vary with time and parameters such as temperature and humidity).

9. A method of controlling a photo-ionization detector (PID), in which method a sample gas enters (typically diffuses) into an ionization chamber of the PID;
said sample gas in the ionization chamber is irradiated by UV radiation from a UV source;
a first electrode generates a first signal indicative of detected gaseous analyte;
at least one sensor measures at least one condition of the sample gas and generates at least one sensor signal;
a controller receives the first signal and the at least one sensor signal;
the controller outputs a measurement signal determined from the first signal and compensated taking into account the at least one sensor signal.

10. A method according to claim 9, wherein the at least one sensor comprises a humidity and/or a temperature sensor, wherein alternatively or optionally the controller controls operation of the UV source wherein optionally the controller switches the UV source repetitively on and off and optionally regulates the duration of each on period and the time between each on period.

11. A method according to either of claims 9 and 10, wherein during illumination of the UV source the controller regulates the power to the UV source.

12. A method of operating a photo-ionization detector (PID), wherein the PID comprises a UV source and a first electrode for generating a measurement signal, in which method control of the UV source depends on the measurement signal generated at the first electrode.

13. A method of operating a photo-ionization detector (PID), wherein a controller varies the frequency of the current driving the UV source of the PID to facilitate finding the optimum frequency (which can vary with time and parameters such as temperature and humidity).

14. A method of maintenance of a photo-ionization detector (PID), the photo-ionization detector comprising a main body, in which method a replaceable electrode stack module comprising a first electrode is combined with the main body, so that the first electrode can be used to detect gaseous analyte, wherein optionally the replaceable electrode stack module comprises a UV source, wherein as a further option or as an additional option the replaceable electrode stack module comprises a solid state memory, which is used for calibration.

15. A method of operating a photoionization detector (PID) comprising a UV source, an second electrode, a first electrode, and a guard electrode, wherein:
a positive potential is applied to the guard electrode
while the guard electrode is maintained at said positive potential, a first measurement is taken from the first electrode and a first background measurement is taken from the guard electrode;
a negative potential is applied to the guard electrode;
while the guard electrode is maintained at said negative potential, a second measurement is taken from the first electrode and a second background measurement is taken from the guard electrode;
a corrected measurement is produced from said first measurement, said second measurement, said first background measurement and said second background measurement.
